(19) Europäisches Patentamt
European Patent Office
Office européen des brevets

(11) Publication number: **0 095 377**
**B1**

(12) **EUROPEAN PATENT SPECIFICATION**

(45) Date of publication of patent specification: **29.10.86**

(51) Int. Cl.$^4$: **A 01 N 59/12,** A 61 K 9/22 // (A01N59/12, 37:20)

(21) Application number: **83302979.6**

(22) Date of filing: **24.05.83**

(54) **Improvements in or relating to iodophors.**

(30) Priority: **25.05.82 GB 8215203**

(43) Date of publication of application:
**30.11.83 Bulletin 83/48**

(45) Publication of the grant of the patent:
**29.10.86 Bulletin 86/44**

(84) Designated Contracting States:
**AT BE CH DE FR IT LI LU NL SE**

(56) References cited:
**GB-A-1 144 637**
**GB-A-1 441 364**
**US-A-4 069 347**
**US-A-4 113 857**

(73) Proprietor: **Wilson, Edward Sturdy**
**Altahammond 57 Belfast Road**
**Whitehead County Antrim Northern Ireland (GB)**

(72) Inventor: **Wilson, Edward Sturdy**
**Altahammond 57 Belfast Road**
**Whitehead County Antrim Northern Ireland (GB)**

(74) Representative: **Kyle, Diana**
**ELKINGTON AND FIFE High Holborn House**
**52/54 High Holborn**
**London WC1V 6SH (GB)**

**EP 0 095 377 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to iodophor compositions or solutions for topical application, and which have inherent emolliency. The iodophor solutions according to the invention are particularly useful as teat dips in preventing mastitis in cows.

Iodophors are carriers of iodine and are usually complexes of iodine with certain types of surfactants with detergent properties. It is possible for iodine to be taken up in chemical combination by high molecular weight surfactants and water-soluble polymers. The surfactants may be nonionic, cationic or anionic but generally the most efficient and stable iodophors are compounds of nonionic surfactants, such as alkoxylated compounds having a free terminal hydroxyl group on the alkoxy chain. Though the iodine in an iodophor is held in loose chemical combination, part of the iodine is available and retains its bactericidal activity. Iodophors may solubilise up to 25% by weight of iodine of which 70 to 80% may be released as available iodine when a concentrated solution is diluted. Solutions of iodophors can be formulated with acid and the bacterial action of most of them is enhanced by lowering the pH. Increases in temperature increase the bactericidal action of iodophors, but above 43°C they break down with the liberation of iodine.

U.S. Patent No. 4,113,857 describes and claims a method of producing an organic iodophor germicidal composition which is substantially free of iodide contamination. The composition is formed by reacting an organic iodophor-forming compound with an iodine adding agent in the presence of an oxidizing stabilizer. The iodophor-forming compound may be *inter alia* a cationic detergent such as an amino-amide. These iodophors based on cationic surfactants, however, have disadvantages for both industrial and medical applications. In industrial applications, the operator is subject to the dangers of contact dermatitis and, in medical applications, the corrosive nature of the cationic surfactant, as a residue on the skin, cannot be tolerated. In particular, the amino-amide type iodophors of U.S. Patent No. 4113857 would not be suitable for use as teat dips, since they would completely destroy the dermal tissue of cows' teats. Rather such amino-amide type iodophors would only be suitable for sanitising inanimate surfaces against microbial contamination as stated in Example 8 of U.S. Patent No. 4113857.

Solutions of iodophors are conventionally employed in pre-operative skin disinfection and for disinfecting instruments and blankets. They are also used in the food industry and in dairy santitation, in particular, as teat dips for cows.

The use of iodophors has largely replaced the use of steam in the cleansing and sterilisation of work-tops and utensils in the catering and food industries, with consequent savings in energy.

The main disadvantage of know iodophors is that with prolonged use they cause dermatological problems such as cracks or fissures which are thought to result from a loss of skin's natural protective fats by a process of emulsification.

It will be appreciated that nonionic surfactants are powerful fat emulsifiers and have a degreasing effect on the skin. This problem is particularly prevalent when iodophors are used as teat dips in bovine hygiene.

The earliest iodophor teat dips employed the aforementioned alkoxylated compounds as the nonionic surfactant complexing agent and were particularly harsh on teat skin. Continued use of such iodophor preparations resulted in a widespread incidence of teat cracking with concomitant risk of infection due to the harbouring of bacteria in cracks and fissures in the skin of the teats.

The problem of teat cracking was significantly reduced with the advent of the iodophors employing alkoxylated lanolin as a nonionic surfactant. Such iodophors form the subject of British Patent Specification No. 1144637. Alkoxylated lanolin, especially ethoxylated lanolin, has an emollient effect which substantially reduces the incidence of teat cracking.

Experiments have shown that no iodine can be detected on the udder skin approximately one hour after dipping of the udder in a conventional iodophor teat dip. This loss of iodine can be attributed essentially to two factors, the first being a high rate of evaporation of iodine as the film of iodophor attains body temperature and the second being a rapid reduction of iodine to iodide in the presence of oxidizable organic soil. Accordingly, if one uses an iodophor teat dip at the morning and evening milking sessions, there will be a period between the two sessions of about 7—8 hours during which no active iodine will be present on the teat skin with consequent risk of bacterial infection or reinfection. If such infection occurs whilst the dermal tissues are fissured then mastitis normally results.

It is an object of the present invention to provide an iodophor composition which has inherent emollient activity coupled with bactericidal activity.

Accordingly, the invention provides an iodophor composition for topical application comprising a complex of iodine with a quaternary ammonium compound of the general formula I:

$$\left[ L{-}NR_1{-}(CH_2)_n{-}\overset{\displaystyle R_2}{\underset{\displaystyle R_4}{N^{\oplus}}}{-}R_3 \right] X^{\ominus} \qquad I$$

wherein L represents an acyl radical derived from lanolin fatty acid, $R_1$ is hydrogen or a $C_{1-4}$ alkyl group, $R_2$ and $R_3$ each represents a $C_{1-4}$ alkyl group, $R_4$ is an alkyl, aryl, aralkyl, hydroxyalkyl or unsaturated aliphatic hydrocarbon radical, X is a compatible anion and n is an integer of from 2 to 5, in an aqueous medium.

Preferably, the quaternary ammonium compound (Q.A.C.) is a compound of formula I wherein $R_1$ is hydrogen, $R_2$ and $R_3$ independently of each other represent a methyl or an ethyl group, $R_4$ is a $C_{1-4}$ alkyl group or an ar($C_{1-4}$)alkyl group, X is a compatible anion and n=2 or 3.

Further, preferably, X is a halide, nitrate, sulphate, alkylsulphate or alkylphosphate anion.

Most preferably, L represents an acyl radical derived from refined lanolin acid.

An especially preferred compound of formula I is one wherein L is an acyl radical derived from a refined lanolin fatty acid, $R_1$ is hydrogen, $R_2$ and $R_4$ are each methyl, $R_3$ is ethyl, n=3 and X is an ethylsulphate ion *viz* lanolinamidopropyldimethylethylammonium ethosulphate.

Commercially available lanolin Q.A.C.'s are primarily used in the cosmetics industry and tend to be made and used in diol (glycol) solution, for example in propylene glycol as described for example in British Patent Specification No 1590012.

It has now been found that iodophor compositions according to the invention can be readily prepared from lanolin Q.A.C.'s in the absence of any diol compound. Thus, lanolinamidopropyldimethylethylammonium ethosulphate *per se* has been successfully complexed with iodine to form an iodophor composition according to the invention.

Many of the lanolin Q.A.C's of formula I which can be complexed with iodine to form the iodophor compositions according to the invention have a consistency which is similar to that of petroleum jelly. With such lanolin Q.A.C.'s it has been found easier to prepare the iodophor composition if the Q.A.C. is used together with a nonionic surfactant. Any commercially available nonionic surfactant is suitable. However, a particularly suitable nonionic surfactant is TEXOFOR V27 (TEXOFOR V27 is a Trade Mark) which is a polyoxyalkylene condensate of a linear fatty alcohol. Other suitable nonionic surfactants are (i) the nonionics referred to as polyoxyethylene derivatives of sorbitan fatty acid esters or alkyl sorbitan polyoxyethylene marketed under the Trade Marks "Tweens", the most preferred being "Tween 80", also known as polysorbate 80 or sorbitan mono-oleate; and (ii) the nonionics referred to as polyoxyethylenepolyoxypropylene block polymers marketed under the Trade Mark "Pluronics", particularly those having molecular weights ranging from 4,000 to 8,000 with approximately 40—70% of the polyoxyethylene hydrophilic polymer and 60—30%, respectively, of the polyoxypropylene hydrophobic polymer, the most preferred being "Pluronic F127" also known as poloxomer 407. Preferably, the lanolin Q.A.C.(s) should be used in an amount of 25—50% by weight and the nonionic surfactant in an amount of 50—75% by weight of the lanolin Q.A.C.(s)/surfactant mixture.

Another especially preferred compound of formula I is one wherein $R_1$ is hydrogen, $R_2$ and $R_3$ each represent methyl, $R_4$ is benzyl, n=3 and X is a chloride ion.

The invention also provides a method for preparing an iodophor composition which comprises forming a complex of iodine with a quaternary ammonium compound of formula I.

Preferably, the iodophor compositions according to the invention have a titratable iodine value in the range of 1.5—2.5% w/w.

The compounds of formula I are known and can be readily prepared by conventional techniques such as by reaction of a lanolin acid of formula II:

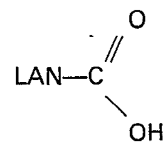
II

wherein LAN represents the fatty acid side chain of a lanolin acid, with a diamine having the formula III:

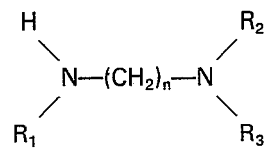
III

wherein $R_1$ is hydrogen or a $C_{1-4}$ alkyl group, $R_2$ and $R_3$ are $C_{1-4}$ alkyl groups and n is an integer from 2 to 5, to form a lanolin amide of formula IV:

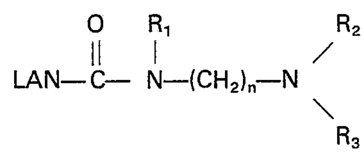
IV

The lanolin amide of formula IV is treated with a suitable quaternizing agent such as an aliphatic halide, aralkyl halide, ethylene chlorohydrin or alkylsulphate so as to obtain the corresponding compound of formula I.

Especially preferred quaternizing agents include alkyl halides such as methyl bromide, ethyl iodide or isopropyl bromide, aryl halides, aralkyl halides such as benzyl chloride, alkylsulphates such as dimethyl or diethyl sulphate or ethylene chlorohydrin. However, any suitable known quaternizing agent can be used.

The lanolin acid of formula II may be any commercially available lanolin fatty acid or mixture of such lanolin fatty acids.

Representative diamines of formula III for preparing preferred compounds of formula IV which are then quaternised to form the corresponding compounds of formula I include: dimethylaminopropylamine, diethylaminopropylamine, dimethylaminoethylamine, and diethylaminoethylamine.

The iodophor compositions according to the invention may comprise a mixture of compounds of formula I.

The iodophor compositions according to the

invention may also include one or more quaternary ammonium halide compound(s) such that the ratio of compounds of formula I to said quaternary ammonium halide compound(s) is not less than 4:1.

The iodophor compositions according to the invention will preferably have a total Q.A.C. concentration in the range 0.5—10% by weight, especially 1—5% by weight.

The iodophor compositions according to the invention may also include a complex of iodine with a nonionic surfactant in addition to the compound of formula I. Suitably said nonionic surfactant may be an alkoxylated lanolin of the type employed in the iodophor compositions of British Patent Specification No. 1144673, especially an ethoxylated lanolin or an alkoxylated compound having a free hydroxyl group on the alkoxy chain such as ethoxylated octyl phenol or ethoxylated nonyl phenol. However, in the compositions according to the invention the compounds of formula I should represent at least 20% by weight of the total surfactant present. The compound of formula I is preferably mixed with any additional nonionic surfactants used before complexing with iodine.

The compounds of formula I have both emollient and antiseptic properties due to bifunctional nature of the molecules of the compound of formula I, unlike conventional quaternary ammonium surfactants.

The iodophor compositions according to the invention are particularly suitable for use as teat or udder dips (hereinafter referred to as teat dips) for cows for the prevention or control of mastitis.

Teat dips according to the invention preferably have an iodine activity or available iodine content of between 0.25—0.75% by weight, especially 0.5% by weight.

The total Q.A.C. concentration of teat dips according to the invention is preferably in the range 5,000—25,000 p.p.m.

The invention further provides a method of disinfecting a locus which comprises applying to said locus an iodophor composition as hereinabove defined.

The iodophor compositions according to the invention have superior emolliency to the iodophor compositions based on alkoxylated lanolin described in British Patent Specification No. 1144637.

The superior emolliency of the compositions according to the invention is considered to be due to their affinity as quaternary ammonium compounds for the keratin of the skin to which they become strongly bonded as do other quaternary ammonium compounds. Nonionic ethoxylated lanolin for example does not have this property.

As quaternary ammonium compounds, the compounds of formula I have biocidal properties in their own right. Accordingly, it will be appreciated, for example, in the case of teat dip, that after the iodine content of the iodophor composition has disappeared as described above due to natural evaporation and reduction of iodine to iodide, a film of the quaternary ammonium compound will remain which has both biocidal and emollient properties, thereby extending the protection afforded by the iodophor composition.

The invention will be further illustrated by the following Examples. The Preparations illustrate the preparation of Q.A.C.'s for complexing with elemental iodine to form iodophor compositions according to the invention.

Preparation 1
Lanolinamidopropyldimethylbenzylammonium chloride

(a) Dimethylaminopropyl lanolin acid amide

One mole (about 340 grams) of warm lanolin fatty acids was poured into a flask which was fitted with a stirrer, thermometer, dropping funnel and reflux condenser. The air in the apparatus was displaced with nitrogen and the contents heated within the range 105—115°C.

Over the next 15 to 20 minutes 130 grams (about 1.3 moles) of dimethylaminopropylamine were added dropwise and the temperature increased to 140°C to 150°C. This temperature range was maintained for some 7 to 8 hours. During the final 4 hours a vacuum was applied which it was possible to reduce to 1 mm Hg during the last hour when the temperature was raised to 160°C.

The reaction mixture was thereafter cooled to yeild the amide of the original lanolin fatty acids.

(b) Quaterization of the dimethylaminopropyl lanolin acid amide

About 200 grams of the dimethylaminopropyl lanolin acid amide were placed in a reaction vessel and heated to 75°C. At this point 62 grams of benzyl chloride were dropped in slowly over a period of 1 hour and the mixture was maintained at 110°C for 3 hours. During the last hour of the reaction the pressure was reduced as before. The mixture was cooled to yield the final product—the quaternary ammonium chloride of the lanolin fatty acids. The final product had the consistency of petroleum jelly and the characteristic odour of a Q.A.C.. Analysis indicated *circa* 78% quaternization.

Preparation 2
Lanolinamidopropyldimethylethylammonium ethosulphate

Dimethylaminopropyl lanolin amide prepared according to Preparation 1—step (a) was heated to 75°C in a reaction vessel and then an equivalent amount of diethylsulphate was added slowly over a period of one hour. The temperature was then raised to 110°C and maintained at that temperature for 3 hours.

During the last half hour of the reaction the pressure was reduced to 1/mmHg.

The mixture was then cooled. The final product had a consistency of petroleum jelly and the

characteristic odour of a Q.A.C. Analysis indicated *circa* 80% quaternization.

Example 1

The final product of Preparation 1 was mixed with a nonionic surfactant TEXOFOR V27 in a ratio of 1:2.

100 g of the Q.A.C./surfactant mixture was mixed with elemental iodine (11.5 g) and the resultant iodophor diluted to 500 ml with deionised water and filled into 50 ml bottles. The iodophor composition so produced gave a titratable iodine value of 1.8% w/w using thiosulphate.

The iodophor composition was diluted with water to give a solution having an iodine activity or available iodine content of 0.5%, which solution was then used as a teat dip.

The teat dip so prepared was used in routine mastitis control in a dairy herd of 50 animals over a period of 8 weeks at the morning and evening milking sessions. Prior to the trial approximately 50% of the animals had some degree of teat cracking. At the end of the trial no animal who was free of teat cracking prior to the trail was found to have developed teat cracking. Furthermore, and more importantly, in the case of approximately 75% of those animals who had teat cracking at the beginning of the trial, no teat cracking was evident at the end of the trial. In the remaining animals there was a considerable improvement in the condition of the teats.

Example 2

The final product of Preparation 2 (5g) was treated with iodine (2 g) and the resultant iodophor diluted to 100 ml with deionised water and filled into bottles. An iodophor was thereby produced having a titratable iodine content of 1.8%. Before use as a teat dip the iodophor solution was diluted with water so as to give an iodine activity of approximately 5 g per litre.

On dipping with the teat dip so prepared it was found that the quaternary ammonium lanolin compound, which has biocidal activity, was still present on the teats after two hours when the iodine had disappeared due to natural evaporation and also by reduction to iodide by organic soil on the teats, which acts as a powerful oxidant. Accordingly, the quaternary ammonium compound provides continued protection to the skin tissue against infection/reinfection until the next milking session.

Example 3

A teat dip was prepared from the following ingredients:

| | Concentration (% w/w) |
|---|---|
| Lanolinamidopropyldimethylethyl ammonium ethosulphate (Product of Preparation 2) | 5.0 |
| TEXOFOR V27 | 10.00 |
| Iodine | 2.1 |
| Water | 82.90 |

Activity: 25,000 p.p.m. Q.A.C.; 18,600 p.p.m. available iodine.

The concentration solution (1 part) was then diluted with water (2 parts) to prepare a teat dip ready for use.

Activity of teat dip: 8,333 p.p.m. Q.A.C.; 6,170 p.p.m. available iodine.

The teat dip was found to be stable for at least 24 hours in an open container.

Example 4

A teat dip was prepared from the following ingredients:

| | Concentration (% w/w) |
|---|---|
| Lanolinamidopropyldimethylbenzylammonium chloride (Product of Preparation 1) | 5.0 |
| TEXOFOR V27 | 10.00 |
| Iodine | 2.1 |
| Water | 82.9 |

The concentrated solution (1 part) was then diluted with water (2 parts) to prepare a teat dip ready for use.

Activity of teat dip: 16,600 p.p.m. Q.A.C.; 6,170 p.p.m. available iodine.

Example 5

A teat dip was prepared from the following ingredients:

| | Concentration (% w/w) |
|---|---|
| Lanolinamidopropyldimethylethylammonium ethosulphate (Product of Preparation 2) | 2.5 |
| Empigen BAC* | 0.5 |
| TEXOFOR V27 | 10.0 |
| Iodine | 2.1 |
| Water | 81.9 |

*BAC=Benzalkonium chloride (Empigen BAC is a Trade Mark)

The concentrated solution (1 part was then diluted with water (2 parts) to prepare a teat dip ready for use.

Activity of teat dip: 10,000 p.p.m. Q.A.C.; 6,170 p.p.m. available iodine.

Bactericidal efficiency of lanolinamidopropyldimethylethylammonium ethosulphate containing iodophor teat dip against *Staphylococcus aureus* (NCTC 6571) on excised cows' teats

Laboratory tests were carried out to demonstrate that the lanolinamidopropyldimethylethylammonium ethosulphate containing iodophor teat dip would exert bactericidal activity on cows' teats 15 hours after application.

Methods

For convenience excised cows' teats were used in preference to working with live animals. The teats were collected from recently slaughtered cows and prepared by the method recommended by Philpot *et al* (1978) J. Dairy Sci *61*: 950—955. The teats were frozen in plastic bags in lots of 10 and thawed immediately prior to the commencement of the experiment.

The stock culture of *Staphylococcus aureus* (NCTC 6571) was grown in Brain Heart Infusion Broth (Oxoid) incubated at 37°C for 18 hours. The suspension contained $4.1\times10^9$ colony forming units (cfu) per millilitre.

The iodophor preparation used contained:

5.0% Lanolinamidopropyldimethylethylammonium ethosulphate
10.0% Synperonic NP9 (Synperonic NP9 is a Trade Mark)
2.1% Iodine (available Iodine 1.85%)

At the start of the experiment 32 arbitrarily selected teats were suspended on stainless steel hooks from elevated metal rods (16 test and 16 control teats). The selection of test and control teats was statistically randomised. The teat dip preparation was applied to each of the test teats by immersion to a depth of approximately 4 cm. Control teats were similarly exposed to distilled water. The teats were maintained at ambient temperatures (21±2°C) for a period of 15 hours.

At the end of this time period each teat was momentarily dipped to a depth of 2.5 cm in the stock *Staphylococcus* culture. The teats were allowed to dry for a period of 45 minutes. At the end of this time each teat was swabbed for 25 seconds with a neutralising solution of 8% (w/v) Sodium thiosulphate, 0.5% v/v Tween 80 and 0.5 (v/v) lecithin. This swab was discarded. A second swab, moistened in the neutralising solution, was then rubbed over the lower 1.5 cm of each teat for a period of 20 seconds. After use the swabs were snapped off into separate 4 ml aliquots of a solution of 0.1% (v/v) thiosulphate, 0.1% (v/v) Tween 80 and 0.1% (v/v) lecithin in Universal bottles.

The swabs in neutralising solution were immediately shaken at a constant high speed for a period of 5 minutes on a Stuart Flask Shaker. Decimal dilutions were prepared for each swab/neutraliser sample. Pour plates were prepared over a range of dilutions using Blood Agar Base No. 2 (Oxoid) supplemented with 7% Bovine serum (Oxoid) as the culture medium. After incubation at 37°C for 18 hours the number of *Staphylococcus* colonies was determined by direct counting.

Results

| | Mean count of *S aureus* recovered (cfu per ml) | |
|---|---|---|
| Control | $6.59\times10^6\pm3.3\times10^6$* | |
| Iodophor containing lanolinamidopropyldimethylethylammonium ethosulphate | 39.4 | 66.5* |

*Standard Deviation

The results show that 15 hours after application the lanolinamidopropyldimethylethylammonium ethosulphate containing iodophor preparation still shows good bactericidal properties against *Staphylococcus aureus* giving greater than log 5 reduction in this test situation.

Bactericidal efficiency of Lanolinamidopropyldimethylethylammonium ethosulphate contain iodophor teat dip

Laboratory tests were carried out to determine the bactericidal efficiency of a Lanolinamidopropyldimethylethylammonium ethosulphate containing iodophor teat dip against the following bacteria:—

| | |
|---|---|
| 1. *Staphylococcus aureus* | NCTC 6571 |
| 2. *Escherichia coli* | NCTC 10418 |
| 3. *Pseudomonas aeruginosa* | NCTC 10662 |
| 4. *Streptococcus agalactiae* | Fermentation type IV (V.R.L.) |

Method

Stock cultures of the bacteria were grown in Brain Heart Infusion broth (Oxoid) incubated at 37°C for 18 hours. The stock cultures were diluted to give working suspensions containing approximately 108 cells per ml.

Bacterial concentrations of working suspensions and numbers of surviving bacteria after trial were estimated by culturing on Blood Agar Base No 2 (Oxoid) supplemented with 7% Bovine serum (Oxoid). Trials were carried out in sterile test-tubes at a temperature of 37°C. Testing was carried out as follows:

Bacterial suspensions were treated with the lanolinamidopropyldimethylethylammonium ethosulphate containing iodophor for a period of 16 hours after which time the preparations were neutralised.

Inactivation of disinfectant after the required contact time was achieved using a neutralizing solution containing 2.0% Lecithin, 2.0% Tween 80, 0.5% Sodium thiosulphate and 0.1% peptone.

The results are shown in Table 1.

The results show that the lanolinamidopropyldimethylethylammonium ethosulphate containing iodophor teat dip has excellent bactericidal activity against the bacteria tested.

The trial shows that the lanolinamidopropyldimethylethylammonium ethosulphate containing iodophor teat dip gives a better than required kill even at low concentration (0.5% lanolinamidopropyldimethylethylammonium ethosulphate; 0.25% $I_2$).

TABLE 1

| Concentration Lanolinamidopropyldimethylethylammonium ethosulphate & Iodine (Q.A.C.) | ($I_2$) | *Staphylococcus aureus* | *Escherichia coli* | *Pseudomonas aeruginosa* | *Streptococcus agalactiae* |
|---|---|---|---|---|---|
| 0.5% | 0.25% | 6.58 | 8.06 | 8.51 | 6.81 |
| 2.5% | 0.25% | 6.58 | 8.06 | 8.51 | 6.81 |

It should be noted that the quaternary ammonium lanolin derivatives used in the iodophor compositions according to the invention are quite different to quaternary ammonium compounds conventionally used in teat dips such as the quaternary ammonium compounds used in the teat dips of the Applicant's British Patent Specification No. 1554615.

As a comparison, if iodine is complexed with quaternary ammonium compounds of the kind used in the teat dips of British Patent Specification No. 1554615, the teat dip formed from such an iodophor will lose its iodine in the normal way within approximately 1 hour, leaving behind a surfactant film containing a quaternary ammonium compound having no lanolin moiety as part of its structure. Accordingly, such a teat dip will be harsh and have a degreasing action on the skin. Furthermore, the quaternary ammonium compounds will bind to the shin causing irritation even in the presence of ethoxylated lanolin which does not bind to the skin.

Apart from their use as teat dips in the dairy industry, the iodophor compositions according to the invention can also be readily used in skin care; as a pre-operative scrub for surgeons' hands; as a preoperative wash for human skin prior to surgical procedures; and as an active ingredient in antidandruff shampoos. A further use for the iodophor compositions according to the invention would be as a prophylactic measure in swimming pools and leisure centres against transmission of foot infections caused by fungal or viral types of organisms, for example verrucae.

**Claims**

1. An iodophor composition for topical application comprising a complex of iodine with a quaternary ammonium compound of the general formula I:

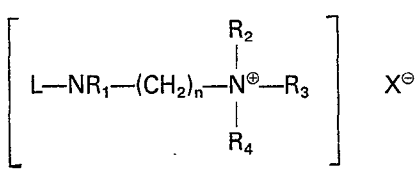

I

wherein L represents an acyl radical derived from lanolin fatty acid, $R_1$ is hydrogen or a $C_{1-4}$ alkyl group, $R_2$ and $R_3$ each represents a $C_{1-4}$ alkyl group, $R_4$ is an alkyl, aryl, aralkyl, hydroxyalkyl or unsaturated aliphatic hydrocarbon radical, X is a compatible anion and n is an integer of from 2 to 5, in an aqueous medium.

2. An iodophor composition according to claim 1, wherein in the compound of formula I $R_1$ is hydrogen, $R_2$ and $R_3$ independently of each other represent a methyl or ethyl group, $R_4$ is a $C_{1-4}$ alkyl group or an ar($C_{1-4}$) alkyl group, X is a compatible anion and n is 2 or 3.

3. An iodophor composition according to claim 1 or 2, wherein in the compound of formula I X is a halide, especially chloride, nitrate, sulphate, alkylsulphate, especially ethylsulphate, or alkylphosphate anion and L represents an acyl radical derived from refined lanolin acid.

4. An iodophor composition according to claim 1, which contains a mixture of compounds of formula I complexed with iodine.

5. An iodophor composition according to any preceding claim, which has a titratable iodine value in the range of 1.5—2.5% w/w.

6. An iodophor composition according to any preceding claim, which includes one or more

quaternary ammonium halide compound(s) such that the ratio of compounds of formula I to said quaternary ammonium halide compound(s) is not less than 4:1.

7. An iodophor composition according to any preceding claim, which contains a total concentration of quaternary ammonium compound(s) in the range 0.5—10% by weight.

8. An iodophor composition according to any preceding claim, which also includes a complex of iodine with a nonionic surfactant, especially an alkoxylated lanolin, an ethoxylated octyl phenol or ethoxylated nonyl phenol or a mixture of said phenols, in an amount of up to 80% of the total surfactant present.

9. An iodophor composition according to any one of the preceding claims, which is in the form of a teat dip and which has an available iodine content in the range of 0.25—0.75% by weight and a total Q.A.C. concentration of 5,000—25,000 p.p.m.

10. An iodophor composition according to any one of claims 1 to 9, for use in the prevention of mastitis in cows.

11. A method of preparing an iodophor composition according to any one of claims 1—9, which comprises forming a complex of iodine with a quaternary ammonium compound of formula I.

12. A method according to claim 12, wherein the quaternary ammonium compound is mixed with a nonionic surfactant, especially a polyoxyalkylene condensate of a linear fatty alcohol, prior to complexing with iodine.

**Patentansprüche**

1. Iodophor-Zusammensetzung für eine topische Anwendung, umfassend einen Komplex aus Jod mit einer quaternären Ammoniumverbindung der allgemeinen Formel (I)

$$\left[ L{-}NR_1{-}(CH_2)_n{-}\underset{R_4}{\overset{R_2}{\overset{|}{\underset{|}{N^{\oplus}}}}}{-}R_3 \right] X^{\ominus} \quad (I)$$

worin bedeuten:

L einen Acylrest, der sich von Lanolinfettsäure ableitet;

$R_1$ Wasserstoff oder eine $C_{1-4}$-Alkylgruppe;

$R_2$ und $R_3$ jeweils eine $C_{1-4}$-Alkylgruppe;

$R_4$ einen Alkyl-, Aryl- Aralkyl-, Hydroxyalkyl- oder ungesättigten aliphatischen Kohlenwasserstoffrest;

X ein verträgliches Anion; und

n eine ganze Zahl von 2 bis 5;

in einem wässrigen Medium.

2. Iodophor-Zusammensetzung gemäss Anspruch 1, worin in der Verbindung der Formel (I) $R_1$ Wasserstoff, $R_2$ und $R_3$ unanhängig voneinander eine Methyl- oder Ethylgruppe, $R_4$ eine $C_{1-4}$-Alkylgruppe oder eine $Ar(C_{1-4})$alkylgruppe, X ein verträgliches Anion und n 2 oder 3 bedeuten.

3. Iodophor-Zusammensetzung gemäss Anspruch 1 oder 2, worin in der Verbindung der Formel (I) X ein Halogenid-, insbesondere Chlorid-, Nitrat-, Sulfat-, Alkylsulfat-, insbesondere Ethylsulfat-, oder Alkylphosphatanion ist und L einen Acylrest, der sich von raffinierter Lanolinsäure ableitet, ist.

4. Iodophor-Zusammensetzung gemäss Anspruch 1, die eine Mischung einer Verbindung der Formel (I) unter Komplexbildung mit Jod enthält.

5. Iodophor-Zusammensetzung gemäss einem der vorhergehenden Ansprüche mit einer titrierbaren Jodzahl im Bereich von 1,5 bis 2,5% G/G.

6. Iodophor-Zusammensetzung gemäss einem der vorhergehenden Ansprüche, welche eine oder mehrere quaternäre Ammoniumhalogenid-Verbindung(en) einschliesst, so dass das Verhältnis der Verbindungen der Formel (I) zu der oder den quaternären Ammoniumhalogenid-Verbindung(en) nicht weniger als 4:1 beträgt.

7. Iodophor-Zusammensetzung gemäss einem der vorhergehenden Ansprüche, welche eine Gesamtkonzentration an quaternärer Ammonium-Verbindung oder quaternären Ammonium-Verbindungen im Bereich von 0,5 bis 10 Gew.% enthält.

8. Iodophor-Zusammensetzung gemäss einem der vorhergehenden Ansprüche, welche ausserdem einen Komplex aus Jod mit einem nicht-ionischen oberflächenaktiven Mittel, insbesondere einem alkoxylierten Lanolin, einem ethoxylierten Octylphenol oder einem ethoxylierten Nonylphenol oder einer Mischung der Phenole in einer Menge von bis zu 80% des gesamten vorhandenen, oberflächenaktiven Mittels einschliesst.

9. Iodophor-Zusammensetzung gemäss einem der vorhergehenden Ansprüche in Form einer Zitzen-Eintauchlösung mit einem Verfügbaren Jodgehalt im Bereich von 0,25 bis 0,75 Gew.% und einer Gesamt-Q.A.C.-Konzentration von 5.000 bis 25.000 ppm.

10. Iodophor-Zusammensetzung gemäss einem der Ansprüche 1 bis 9 für die Verwendung zur Verhinderung von Mastitis bei Kühen.

11. Verfahren zur Herstellung einer Iodophor-Zusammensetzung gemäss einem der Ansprüche 1 bis 9, dadurch gekennzeichnet, dass man einen Komplex aus Jod mit einer quaternären Ammoniumverbindung der Formel (I) bildet.

12. Verfahren gemäss Anspruch 12, dadurch gekennzeichnet, dass die quaternäre Ammonium-Verbindung mit einem nicht ionischen oberflächenaktiven Mittel, insbesondere einem Polyoxyalkylen-Kondensat eines linearen Fettalkohols, vor dem Komplexbilden mit dem Jod vermischt wird.

**Revendications**

1. Composition d'iodophore pour l'application

topique, comprenant du complexe d'iode avec un composé d'ammonium quaternaire de la formule générale I:

$$\left[ L-NR_1-(CH_2)_n-\overset{\displaystyle R_2}{\underset{\displaystyle R_4}{\overset{|}{\underset{|}{N^{\oplus}}}}}-R_3 \right] X^{\ominus} \qquad I$$

dans laquelle L représente un radical acyle provenant d'un acide gras de lanoline $R_1$ représente l'hydrogène ou un groupe alkyle en $C_1$—$C_4$, $R_2$ et $R_3$ représentent chacun un groupe alkyle en $C_1$—$C_4$, $R_4$ représente un radical alkyle, aryle, aralkyle, hydroxyalkyle ou hydrocarburé aliphatique insaturé, X représente un anion compatible et n est un nombre entier de 2 à 5, dans un milieu aqueux.

2. Composition d'iodophore suivant la revendication 1, caractérisée en ce que dans le composé de la formule I, $R_1$ représente de l'hydrogène, $R_2$ et $R_3$ représentent indépendamment l'un de l'autre un groupe méthyle ou éthyle, $R_4$ représente un groupe alkyle en $C_1$—$C_4$ ou un groupe ar($C_1$—$C_4$) alkyle, X représente un anion compatible et n est égal à 2 ou 3.

3. Composition d'iodophore suivant l'une ou l'autre des revendications 1 et 2, caractérisée en ce que dans le composé de la formule I, X représente un anion d'halogénure, en particulier de chlorure, de nitrate, de sulfate, d'alkylsulfate, en particulier d'éthylsulfate ou d'alkylphosphate et L représente un radical acyle provenant d'un acide de lanoline raffinée.

4. Composition d'iodophore suivant la revendication 1, caractérisée en ce qu'elle contient un mélange de composés de la formule I complexés par de l'iode.

5. Composition d'iodophore suivant l'une quelconque des revendications précédentes caractérisée en ce qu'elle présente un indice d'iode titrable de l'ordre de 1,5 à 2,5% en poids/poids.

6. Composition d'iodophore suivant l'une quelconque des revendications précédentes, caractérisée en ce qu'elle comprend un ou plusieurs composé(s) d'halogénure d'ammonium quaternaire de telle sorte que le rapport des composés de la formule I à ce ou ces composés d'halogénure d'ammonium quaternaire ne soit pas inférieur à 4/1.

7. Composition d'iodophore suivant l'une quelconque des revendications précédentes, caractérisée en ce qu'elle contient une concentration totale de composé(s) d'ammonium quaternaire de l'ordre de 0,5 à 10% en poids.

8. Composition d'iodophore suivant l'une quelconque des revendications précédentes, caractérisée en ce qu'elle comprend également un complexe d'iode avec un agent tensio-actif non ionique, en particulier une lanoline alcoxylatée, un octyl phénol éthoxylé ou un nonyl éthoxylé ou encore un mélange de ces phénols, en une quantité allant jusqu'à 80% de l'agent tensio-actif total présent.

9. Composition d'iodophore suivant l'une quelconque des revendications précédentes, caractérisée en ce qu'elle est sous la forme d'un bain pour mamelle et en ce qu'elle a une teneur en iode disponible de l'ordre de 0,25 —0,75% en poids et une concentration totale Q.A.C. de 5.000—25.000 p.p.m.

10. Composition d'iodophore suivant l'une quelconque des revendication 1 à 9, utilisable dans la prévention de la mastite chez les vaches.

11. Procédé de préparation d'une composition d'iodophore suivant l'une quelconque des revendications 1 à 9, caractérisé en ce qu'il comprend la formation d'un complexe d'iode avec un composé d'ammonium quaternaire de la formule I.

12. Procédé suivant la revendication 12, caractérisé en ce que l'on mélange le composé d'ammonium quaternaire avec un agent tensio-actif non ionique, en particulier un produit de condensation de polyoxyalkylène et d'un alcool gras linéaire, avant la complexation par l'iode.